# EUROPEAN PATENT APPLICATION

(11) **EP 4 163 364 A1**
(43) Date of publication of application: **12.04.2023**
(21) Application number: 21821101.9
(22) Date of filing: 07.06.2021
(51) Int. Cl.: C12N 5/071, C12M 3/00

(54) **CELL CULTURE METHOD**

(30) Priority: 08.06.2020 JP 2020099128
(71) Applicant: National University Corporation Tokyo Medical and Dental University, Tokyo 113-8510 (JP); Takeda Pharmaceutical Company Limited, Osaka-shi, Osaka 541-0045 (JP)
(72) Inventor: TAKEBE, Takanori, Tokyo 113-8510 (JP); SAIKI, Norikazu, Tokyo 113-8510 (JP)
(74) Representative: Jones, Nicholas Andrew
(86) International application number: PCT/JP2021/021478
(87) International publication number: WO 2021/251312

(57) **Abstract**

The present invention provides a culture method capable of maturing an organoid through long-term culture, and suitable for producing a conformational organ. The culture method of the present invention is a method for culturing an organoid and/or cells constituting an organ immobilized in a chamber with a culture fluid perfused, and the culture fluid is perfused in such a manner as to generate a turbulent flow in the chamber.

## Description

### Technical Field

The present invention relates to a method for culturing an organoid and/or cells constituting an organ. More particularly, the present invention relates to a method for culturing an organoid and/or cells constituting an organ with a culture fluid perfused.

### [Background Art]

Aiming to develop medicaments for diseases of various organs including liver and pancreas and realize regenerative medicine, research and development have been conducted to produce a cell structure obtained by self-assembly of cultured cells to reproduce a three-dimensional structure of each organ or a complicated structure of a blood vessel, a bile duct or another vessel, namely, an organoid. As an advanced cell structure of such an organoid, an anlage of an organ formed at an early stage of development, namely, an organ bud (such as a germ corresponding to an organ bud of liver) can be produced by culturing a combination of specific types of cells including an undifferentiated cell. Such an organoid that can be designated also as a "mini-organ" is, as compared with conventional cells and the like of an organ each singly cultured, highly useful because the organoid can be used as an evaluation system closer to a living body in evaluation of medicinal effect or toxicity, and in addition, can be used in a transplant operation or production of a plasma protein.

Various organoids are generally produced by culture performed under an environment where a culture fluid (liquid medium) is static (static culture), but culture performed under an environment where a culture fluid is perfused (perfusion culture) has been also proposed. For example, Non Patent Literature 1 describes that a glomerule blood vessel is developed by perfusion culture of a kidney organoid. Besides, Non Patent Literature 2 describes that long-term culture is performed with keeping balance between nutrient supply and respiration by statically placing a biological tissue section (suprachiasmatic nucleus (SCN) of brain tissue) on a culture fluid permeable membrane with a microfluidic device, and controlling perfusion of a culture fluid to keep a liquid covering the tissue at an appropriate amount. Non Patent Literature 3 describes that when a lung epithelial cell is perfusion cultured on an air-liquid interface (ALI), growth and differentiation of the cell are accelerated than in static culture. It is general, in such perfusion culture of an organoid, a tissue and the like, that a culture fluid is perfused at a constant rate to obtain a "laminar flow", and that application of shear stress to the organoid, the tissue, and the like is avoided as much as possible.

### Citation List

### Non Patent Literature

Non Patent Literature 1: Homan, K. A. et al., Flow-enhanced vascularization and maturation of kidney organoids in vitro, Nat Methods 16, 255-262 (2019) doi:10.1038/s41592-019-0325-y
Non Patent Literature 2: Ota, N. et al., A Microfluidic Platform Based on Robust Gas and Liquid Exchange for Long term Culturing of Explanted Tissues, Analytical Sciences, Oct 2019, Vol.35
Non Patent Literature 3: Long term Culturing of Explanted Tissues, Analytical Sciences, Oct 2019, Vol.35

### Summary of Invention

### Technical Problem

It was not easy to produce an organoid matured through long-term culture, and further a conformational organ by static culture conventionally generally employed. For example, when a liver organoid is cultured by static culture for a long period of time, it is difficult to retain matured hepatocytes, cell death is caused by the culture for about 2 weeks, and there arises a problem that a cell density is lowered to form a gap between cells.

An object of the present invention is to provide a culture method capable of maturing an organoid by long-term culture, and suitable for producing a conformational organ.

### Solution to Problem

The present inventors have found that an organoid or a conformational organ containing mature cells and having a higher density can be produced by intermittently increasing a flow rate of a culture fluid (liquid medium) to be perfused, namely, by intermittently increasing the flow rate of the culture fluid in a chamber, to load a "turbulent flow" in a transient transition state to the organoid and/or the cells constituting an organ, resulting in accomplishing the present invention.

Specifically, the present invention provides the following [1] to [15] for solving the above-described problems:
[1] A culture method for culturing an organoid and/or cells constituting an organ immobilized in a chamber with a culture fluid perfused, wherein the culture fluid is perfused in such a manner as to generate a turbulent flow in the chamber.
[2] The culture method according to item 1, wherein the culture fluid is perfused in such a manner as to have time of transition from a laminar flow to a turbulent flow, time of generation of a turbulent flow and time of transition from a turbulent flow to a laminar flow in the chamber.
[3] The culture method according to item 1 or 2, wherein the organoid and/or the cells constituting an organ is immobilized in a state embedded in a matrix.
[4] The culture method according to any one of items 1 to 3, wherein a matrix in which the organoid and/or the cells constituting an organ is embedded is immobilized in the chamber in a state suspended from a breathable membrane.
[5] The culture method according to any one of items 1 to 4, wherein the organoid has a vascular structure, and/or the cells constituting an organ coexists with vascular cells.
[6] The culture method according to any one of items 1 to 5, wherein the organoid is a liver or kidney organoid, and/or the organ is liver or kidney.
[6a] The culture method according to any one of items 1 to 5, wherein the organoid is an organoid of liver, kidney, pancreas, thyroid gland, parathyroid gland, lung, brain, or heart.
[7] A method for producing a conformational organ, comprising a step of performing the culture method according to any one of items 1 to 6.
[8] The culture method according to any one of items 1 to 6, wherein the culture fluid contains a drug.
[9] The culture method according to item 8, wherein the drug is for treatment of a disease involving ischemia.
[9a] The culture method according to item 8, wherein the drug is for treatment of an ischemic disease, an infarction or necrosis disease, or a hemorrhagic disease
[9b] The culture method according to item 8, wherein the drug is for safety evaluation on a side reaction involving ischemia or bleeding.
[10] A medicinal effect evaluation method, comprising a step of performing the culture method according to item 8 or 9.
[10a] A medicinal effect evaluation method, comprising a step of performing the culture method according to item 8, 9, 9a or 9b, and a step of evaluating efficacy and/or safety of the drug against the organoid.
[11] An organoid obtained by the culture method according to any one of items 1 to 6.
[12] A conformational organ obtained by the production method according to item 7.
[13] A medicinal effect evaluation method, comprising a step of adding a drug to a medium of the conformational organ according to item 12, and a step of evaluating a medicinal effect of the drug on the conformational organ.
[13a] A medicinal effect evaluation method, comprising a step of adding a drug to a medium of the conformational organ according to item 12, and a step of evaluating efficacy and/or safety of the drug against the conformational organ.
[14] A culture system, comprising: a chamber capable of holding an organoid and/or cells constituting an organ immobilized therein, and including an inlet and an outlet for perfusing a culture fluid for the organoid and/or the cells constituting an organ; and perfusion means for perfusing the culture fluid, wherein the perfusion means is controlled in such a manner as to generate a turbulent flow in the culture fluid in the chamber.
[15] The system according to item 14, wherein the perfusion means is controlled to perfuse the culture fluid in such a manner as to have time of transition from a laminar flow to a turbulent flow, time of generation of a turbulent flow and time of transition from a turbulent flow to a laminar flow in the chamber.

### Advantageous Effects of Invention

According to a culture method of the present invention, an organoid in which mature cells are distributed at a higher density than in a conventional technique can be easily produced, and a conformational organ more excellent than one obtained by a conventional technique can be easily obtained from such an organoid. Besides, in an organoid or a conformational organ obtained by the culture method of the present invention, or an artificial organ utilizing the conformational organ, or in regenerative medicine having the conformational organ transplanted, efficacy or safety of various drugs can be evaluated under conditions where reproducibility of the action of the drugs are regarded to be higher than in a conventional technique.

### Brief Description of Drawings

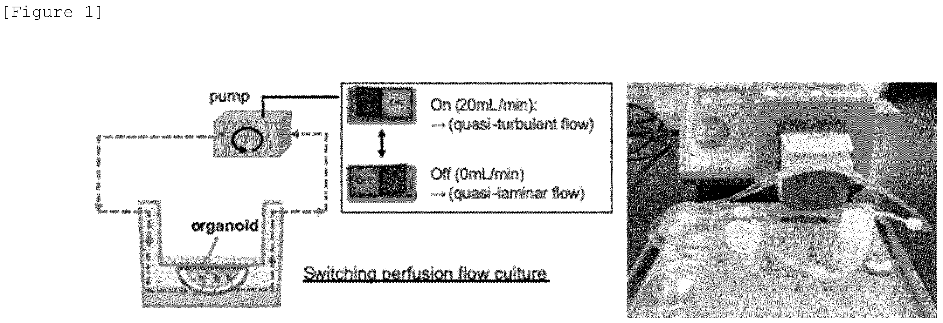

[Figure 1] Figure 1 is a schematic diagram of a culture apparatus (culture system of the present invention) constructed in Examples (1 and 2 in common).

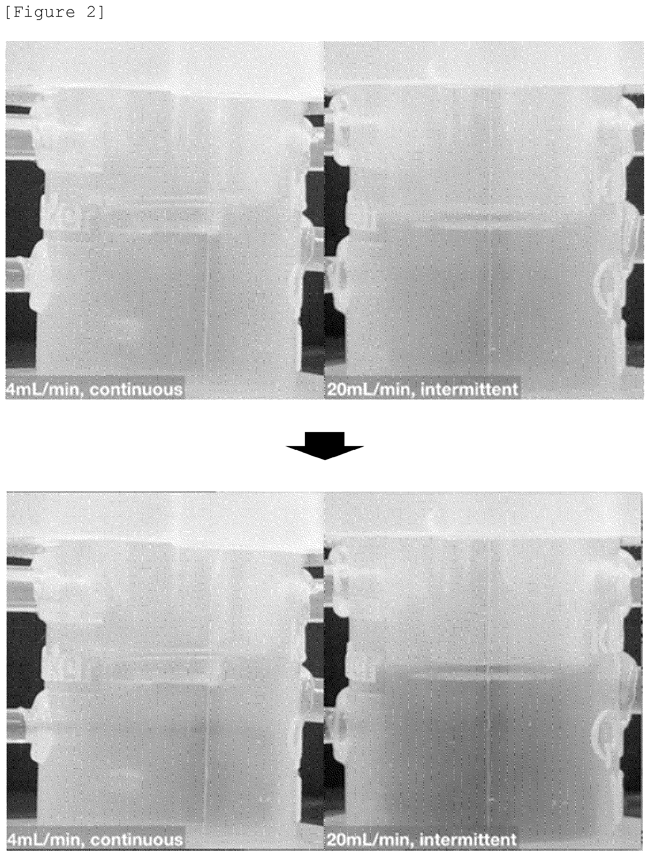

[Figure 2] Figure 2 illustrates images of liquid media held in culture chambers obtained immediately before liquid feed (upper portion) and during the liquid feed (lower portion) under a liquid feed condition where a turbulent flow is generated in Examples (1 and 2 in common) (20 mL/min, intermittent), and a conventional liquid feed condition where a turbulent flow is not generated (4 mL/min, continuous) employed as a reference example in accordance with Non Patent Literature 1. The liquid media are stained with a trypan blue dye. In the liquid feed condition of the reference example, the liquid medium flows into the chamber neatly linearly (namely, in the form of a laminar flow) from an inlet on the left side. On the contrary, in the liquid feed condition of Examples, the liquid medium flows into the chamber swirlingly (namely, in the form of a turbulent flow), and in addition, the liquid medium thus flowed in hits a wall surface of the chamber to generate a turbulent flow in the entire chamber.

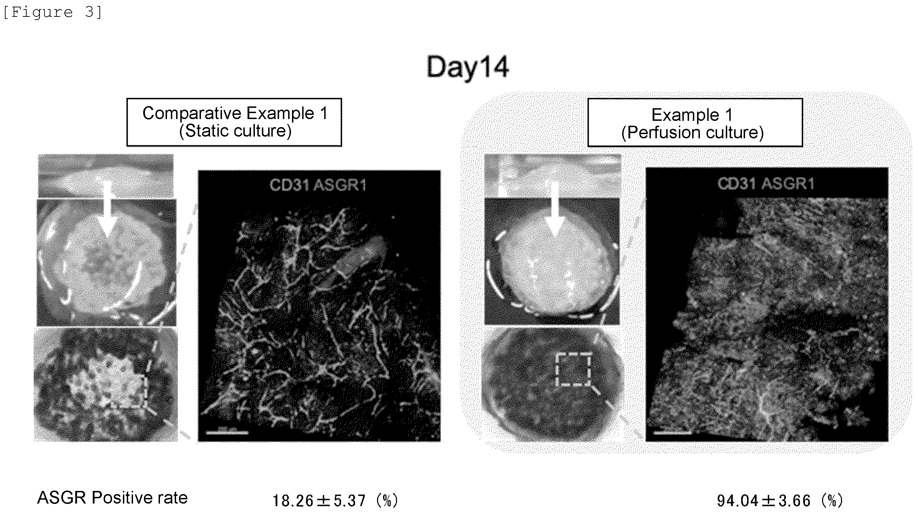

[Figure 3] Figure 3 illustrates fluorescence images of optical microscopic images (side surface and upper surface), stained nuclear images, and immunostained images (double stained images with ASGR1 and CD31, which are respectively in red and green in a color image) of an organoid obtained by perfusion culture of Example 1 (culture method of the present invention) (right portion), and an organoid obtained as a control by static culture of Comparative Example 1 (left portion), and ASGR1 positive rates (average ± standard deviation, average obtained with n = 3) calculated based on a fluorescence region area in each fluorescence image.

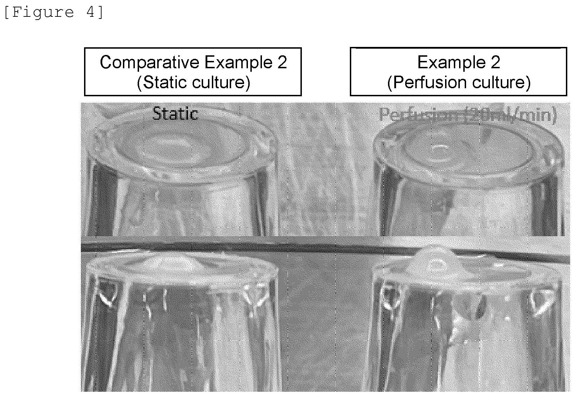

[Figure 4] Figure 4 illustrates observed images of a matrix composition containing an organoid obtained by perfusion culture of Example 2 (culture method of the present invention) (right portion), and a matrix composition containing an organoid obtained as a control by static culture of Comparative Example 2.

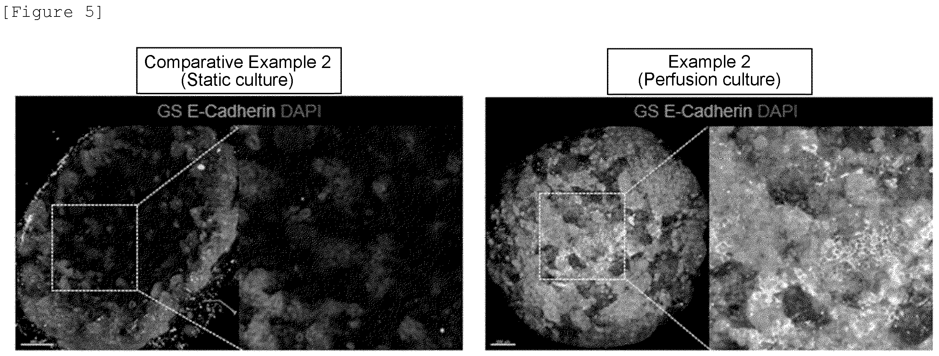

[Figure 5] Figure 5 illustrates immunostained images (trichrome stained images with GS, E-cadherin, and DAPI, which are respectively in red, green, and blue in a color image) of the organoid obtained by the perfusion culture of Example 2 (culture method of the present invention) (right portion), and the organoid obtained as a control by the static culture of Comparative Example 2 (left portion). In the immunostained image of Example 2 (right portion), GS and E-cadherin, that is, mature stem cell markers, are found to express, and the cell density is rich, but in the immunostained image of Comparative Example 2 (left portion), these markers are not found to express, and cell dropout is found particularly in a central portion of the organoid.

### Description of Embodiments

### - Method for Culturing Organoid or the like -

A culture method of the present invention is a method for culturing an organoid and/or cells or a tissue constituting an organ (herein, generically referred to as an "organoid or the like") immobilized in a chamber with a culture fluid perfused, in which the culture fluid is perfused in such a manner as to generate a turbulent flow in the chamber.

The term "chamber" refers to a portion provided in a culture vessel (cell culture device), and having a size capable of holding an organoid or the like to be cultured, and a structure capable of immobilizing the organoid or the like. In general, the chamber is provided with an inlet and an outlet for the culture fluid to be perfused, and is constructed as a portion having larger width and height than a culture fluid passage communicating with the inlet and the outlet. The culture fluid flowing in through the inlet contacts with the immobilized organoid or the like, and then flows out through the outlet. A general culture vessel for perfusion culture of a cell is commercially available, and a similar culture vessel can be used in the present invention.

### <Turbulent Flow and Laminar Flow>

In the present invention, that a culture fluid generates a "turbulent flow" in a chamber refers to that a flow of the culture fluid within the chamber is not uniform but it can be visually confirmed that a vortex is generated in the culture fluid within the chamber, particularly around an immobilized organoid or the like, or around a matrix having the organoid or the like embedded therein, or in the vicinity of the inlet or a wall surface of the chamber, preferably at a higher level than a prescribed level (of, for example, a frequency per unit time, a size or the like). On the other hand, the term "laminar flow" relating to one preferable embodiment of the present invention described below refers to that a flow of the culture fluid within the chamber is substantially uniform, and that it can be visually confirmed that only a vortex at a lower level than the prescribed level is generated, or preferably no or substantially no vortex of the culture fluid is generated within the chamber, particularly around the immobilized organoid or the like, or the matrix having the organoid or the like embedded therein, or in the vicinity of the inlet or the wall surface of the chamber. If the perfusion of the culture fluid within the chamber can be modeled by using a pipe flow, an open channel flow or the like, and thus a Reynolds number can be calculated based on a characteristic length (a hydraulic diameter, for example, a diameter obtained, in a pipe flow, assuming that a cross-section vertical to the perfusion direction is circular), a characteristic flow velocity, a density, a viscosity coefficient and the like, the Reynolds number is compared with a prescribed reference (for example, 2,300) to determine that the flow is a turbulent flow when the number is larger, and is a laminar flow when smaller.

Means for generating a turbulent flow in the chamber is not especially limited, and for example, a turbulent flow can be generated within the chamber by setting a flow rate (for example, a value expressed in a unit of "mL/min") or a flow velocity (a value calculated in accordance with the flow rate/the cross-sectional area of the chamber or the like) of the culture fluid within the chamber to be higher than a prescribed level so as to cause the culture fluid comparatively strongly hit the wall surface of the chamber. Alternatively, a structure (a projection or the like) for generating a turbulent flow may be provided within the chamber, so that the culture fluid can hit the structure to generate a turbulent flow.

During a period when the organoid or the like immobilized in the chamber is cultured with the culture fluid perfused, a turbulent flow may be generated without changing the condition, or may be generated with the condition changed.

In one preferable embodiment of the present invention, the culture fluid is perfused in such a manner as to have (i) time of transition from a laminar flow to a turbulent flow in the chamber, (ii) time of generation of a turbulent flow, and (iii) time of transition from a turbulent flow to a laminar flow. In this embodiment, a turbulent flow is not always caused in the culture fluid within the chamber, but (i) a transition state from a laminar flow to a turbulent flow, (ii) a turbulent flow generation state, (iii) a transition state from a turbulent flow to a laminar flow, and (iv) a laminar flow (optionally, static) state are repeated. Time lengths of the states (i) to (iv) are not particularly limited, and can be adjusted, for example, in accordance with the flow rate or the flow velocity (hereinafter, generically referred to as the "flow rate or the like") of the culture fluid to be perfused. Specifically, the time length of the state (i) can be adjusted in accordance with time for increasing the flow rate from a prescribed flow rate or the like for generating a laminar flow (hereinafter referred to as the "first flow rate or the like") to a prescribed flow rate or the like for generating a turbulent flow (hereinafter referred to as the "second flow rate or the like), or in accordance with time when liquid feed means described below, such as a pump or the like capable of automatically changing the flow rate or the like, is switched from setting for the first flow rate or the like to setting for the second flow rate or the like, and the flow rate within the passage and the chamber is accordingly changed. Similarly, the time length of the state (ii) can be adjusted in accordance with time when the second flow rate or the like is retained, the time length of the state (iii) can be adjusted in accordance with time when the second flow rate or the like is lowered to the first flow rate or the like, and the time length of the state (iv) can be adjusted in accordance with time when the first flow rate or the like is retained, or these time lengths can be adjusted in accordance with the corresponding setting or the like in the liquid feed means. For example, in a culture vessel including a given chamber, a flow rate or the like in a general range usually set to generate a "laminar flow" within the chamber can be dealt with as the "first flow rate or the like", and a higher flow rate or the like that is not conventionally generally employed can be dealt with as the "second flow rate or the like".

### <Organoid or the like>

The term "organoid" refers to an artificially created tissue structure (three-dimensional structure) similar to an organ or a tissue. In the present invention, the term "organoid" is used as a generic term for organoids of various organs and tissues, and encompasses not only organoids of organs or tissues at the mature stage but also structures designated as an "organ bud" or an "anlage" formed at an early stage of complication.

It is noted that the present invention is applicable to not only to an "organoid" but also to a "cancer organoid" (see, for example, JP 2018-110575 A). Specifically, when a "cancer organoid" is immobilized in the chamber instead of an "organoid" to culture the cancer organoid with the culture fluid perfused to generate a turbulent flow within the chamber in the same manner as in the culture method of the present invention, a cancer organoid in which cancer cells are distributed at a higher density than in a conventional one can be produced. Accordingly, the term "organoid" used herein can be replaced with "cancer organoid" if necessary, or can be replaced with "organoid" that is a generic term for "organoid" and "cancer organoid". Besides, a "cancer organoid" can be used for performing a "drug evaluation method" of the present invention.

As the "organoid", various types of organoids, such as organoids of liver, pancreas, kidney, heart, lung, spleen, esophagus, stomach, thyroid gland, parathyroid gland, thymus, gonad, brain, spinal cord, skin, and inner ear have been already known (see, for example, https://www.nejm.org/doi/pdf/10.1056/NEJMra1806175, https://www.nature.com/articles/s41568-018-0007-6 and http://www.amsbio.com/brochures/organoid-culture-handbook.pdf). Any of various organoids can be used in accordance with the purpose of performing the culture method of the present invention, and for example, organoids of liver, kidney, pancreas, thyroid gland, parathyroid gland, lung, brain, and heart are preferable organoids of the present invention. These organoids can be used particularly for performing a medicinal effect evaluation method for a drug for treating an ischemic disease or the like described below.

A basic embodiment of a method for producing various organoids, for example, necessary cells and culture conditions, is known, and an embodiment of the culture method of the present invention can be basically performed according to a known method for producing an organoid. For example, a method for co-culturing (A) cells constituting an organ, (B) vascular endothelial cells, and (C) mesenchymal cells (preferably mesenchymal stem cells), as described in WO2013/047639, WO2015/129822, and the like, is preferred, also in the culture method of the present invention, as a method for producing an organoid.

The term "cell or tissue constituting an organ" used herein refers to a cell that is used for producing an organoid (together with another cell such as a vascular endothelial cell or a mesenchymal cell if necessary) and is necessary for constructing at least a part of the organoid, or a tissue containing such a cell. The "cell or tissue constituting an organ" may be one at a stage of a simple cell aggregate (spheroid) that does not have a structure or a characteristic as that of an organoid, or may be a cell or tissue isolated from an individual (such as a patient-derived biopsy sample). Various "cells or tissues constituting organs" are known, and a cell can be selected in accordance with the type of an organoid. It is noted that "cells constituting an organ" to be immobilized in the chamber can be replaced with "cells or a tissue for producing an organoid containing at least cells or a tissue constituting an organ" if necessary.

The "cell constituting an organ" encompasses (A1) a parenchymal cell constituting an organ or a tissue, and (A2) a non-parenchymal cell constituting an organ or a tissue. Besides, each of the parenchymal cell (A1) and the non-parenchymal cell (A2) encompasses (m) a differentiated, mature, or terminally differentiated cell with a prescribed functionality as a parenchymal cell or non-parenchymal cell (herein, simply referred to as a "differentiated cell"), and (n) a cell capable of differentiating into a parenchymal cell or a non-parenchymal cell, or destined (committed) for differentiation but undifferentiated, or at a stage of a stem cell or a progenitor cell that does not sufficiently have a prescribed functionality as a parenchymal cell or a non-parenchymal cell (herein, simply referred to an "undifferentiated cell").

As the "cell constituting an organ", at least one cell selected from the group consisting of a differentiated cell of a parenchymal cell, an undifferentiated cell of a parenchymal cell, a differentiated cell of a non-parenchymal cell, and an undifferentiated cell of a non-parenchymal cell, preferably a combination of two or more cells capable of forming an organoid (preferably an organ bud) can be used.

Examples of the differentiated "parenchymal cell" include a hepatocyte of liver, a pancreatic endocrine cell (for example, an α cell, a β cell, a δ cell, an ε cell, and a PP cell) and a pancreatic duct epithelial cell of pancreas, a renal tubular epithelial cell and a glomerular epithelial cell of kidney, a pulmonary alveolar epithelial cell of lung, a cardiac cardiomyocyte, an intestinal epithelial cell, a nerve cell and a glial cell of brain, and a nerve cell and a Schwann cell of spinal cord.

Examples of the differentiated "non-parenchymal cell" include a hepatic sinusoidal endothelial cell, a hepatic stellate cell, and a Kupffer cell of liver, a pancreatic stellate cell and a pancreatic microvascular endothelial cell of pancreas, a renal glomerular endothelial cell of kidney, a pulmonary artery endothelial cell and a lung fibroblast of lung, a cardiac microvascular endothelial cell, an aortic endothelial cell, a coronary endothelial cell, and a cardiac fibroblast of heart, an intestinal microvascular endothelial cell of intestinal tract, a cerebral microvascular endothelial cell of brain, a vascular pericyte, a choroid plexus endothelial cell, and a cerebrovascular adventitial fibroblast.

Examples of the "undifferentiated cell" capable of differentiating into a parenchymal cell or a non-parenchymal cell include cells capable of differentiating into ectodermal organs such as brain, spinal cord, adrenal medulla, epidermis, hair, nail or skin glands, sensory organs, peripheral nerves, and lens; cells capable of differentiating into mesodermal organs such as kidney, ureter, heart, blood, reproductive glands, adrenal cortex, muscles, skeleton, dermis, connective tissue, and mesothelium; and cells capable of differentiating into endodermal organs such as liver, pancreas, intestinal tract, lungs, thyroid glands, parathyroid glands, and urinary tract.

The cells constituting an organ may be a primary cultured cell, may be a subcultured cell (established cell), or may be a cell obtained through differentiation such as an ES cell or an iPS cell.

The organoid and/or the cells constituting an organ (organoid or the like) may be derived from a human or derived from an animal except for a human, for example, mammals such as a mouse, a rat, a dog, a pig, and a monkey. In other words, the organoid or the like may be derived from a human, or may be derived from an animal except for a human. From the viewpoint of detecting a drug causing drug addiction, which is difficult to find in conventional animal experiments or human cell tests performed in the development of human medicaments, the organoid is preferably derived from a human. The same applies to the other cells (such as a vascular endothelial cell and a mesenchymal cell) to be used together with the cells constituting an organ if necessary for producing an organoid.

The type of the organ or tissue of the organoid to which the culture method of the present invention is applied is not especially limited, but may accord with the use. Description will be given below by using a liver organoid and a kidney organoid as specific examples, but the present invention can be similarly practiced on organoids of pancreas, thyroid gland, parathyroid gland, lung, brain, heart and the other organs.

In one preferable embodiment of the present invention, the organoid is a liver organoid (preferably a liver bud), and the cells constituting an organ is cells constituting liver (parenchymal cells and/or non-parenchymal cells of liver). A liver organoid (liver bud) can be produced, in accordance with a method described in, for example, WO2013/047639, WO2015/129822 or the like, by co-culturing liver endoderm cells (corresponding to an undifferentiated cell of a liver parenchymal cell), vascular endothelial cells, and mesenchymal cells (preferably mesenchymal stem cells).

In the "cell constituting liver", the parenchymal cell of liver (hepatocyte) encompasses both concepts of a hepatocyte that has been differentiated (differentiated hepatocyte) and a cell destined to differentiate into a hepatocyte but not yet differentiated into a hepatocyte (undifferentiated hepatocyte), that is, so-called a hepatic progenitor cell (such as a hepatic endoderm cell). The differentiated hepatocyte may be a cell collected from a living body (isolated from liver in a living body) or may be a cell obtained by differentiating a pluripotent stem cell such as an ES cell or an iPS cell, a hepatic progenitor cell, or another cell having the ability to differentiate into a hepatocyte. The undifferentiated hepatocyte may be a cell collected from a living body, or may be a cell obtained by differentiating a pluripotent stem cell such as an ES cell or an iPS cell, or another stem cell or progenitor cell. The cell capable of differentiating into a hepatocyte can be produced, for example, according to K. Si-Taiyeb, et al. Hepatology, 51 (1): 297-305 (2010), or T. Touboul, et al. Hepatology. 51 (5): 1754-65 (2010). A method for differentiating, into a hepatocyte, a pluripotent stem cell such as an ES cell or an iPS cell, a hepatic progenitor cell, or another cell having the ability to differentiate into a hepatocyte is known, and an iPS cell can be differentiated, for example, according to a method described in Hepatology, 2010; 51 (1): 297-305, Cell Rep. 2017; 21 (10): 2661-2670, or the like. For both of a cell population collected from a living body and a cell population produced by inducing differentiation of an ES cell, an iPS cell, or the like (particularly, for the latter), a cell population with high purity of a differentiated hepatocyte or a cell population with high purity of an undifferentiated hepatocyte may be used, or a cell mixture containing a differentiated hepatocyte and an undifferentiated hepatocyte at an optional ratio may be used.

It can be determined whether or not a cell is a differentiated hepatocyte by determining whether or not expression of one or more mature hepatocyte markers, such as asialoglycoprotein receptor 1 (ASGR1), glutamine synthase (GS), E-cadherin, α fetoprotein (AFP) as an immature hepatocyte marker (initial liver differentiation marker), albumin (ALB) as an initial liver differentiation marker, retinol-binding protein (RBP4), transthyretin (TTR), and glucose-6-phosphatase (G6PC), are positive. On the other hand, it can be determined whether or not a cell is an undifferentiated hepatocyte by determining whether or not expression of one or more cell markers, such as HHEX, SOX2, HNF4α, AFP, and ALB, are positive (for ALB, whether or not the expression thereof is weakly positive).

The term "vascular endothelial cell" is a term encompassing both concepts of a hemogenic endothelial cell (HEC) and a non-hemogenic endothelial cell (non-HEC). HEC is a vascular endothelial cell capable of producing a hematopoietic stem cell (having hematopoietic ability), and is also designated as a blood cell-producing vascular endothelial cell. On the other hand, non-HEC is a vascular endothelial cell that does not have such hematopoietic ability. When a vascular endothelial cell is used, the resultant organoid can be provided with a fine vascular network.

The vascular endothelial cells may be a cell population with high purity of vascular endothelial cells collected from a living body (such as microvessel endothelial cells (MVECs), liver sinusoidal endothelial cells (LSECs), or umbilical-vein endothelial cells (UVECs)), or may be a cell population with high purity of a vascular endothelial cell obtained by differentiating pluripotent stem cells such as an ES cells or iPS cells, or other cells having the ability to differentiate into vascular endothelial cells.

A method for differentiating, into a hemogenic endothelial cell (HEC), a pluripotent stem cell such as an ES cell or an iPS cell, or another cell having the ability to differentiate into a vascular endothelial cell is known, and an iPS cell can be differentiated, for example, according to a method described in PLoS One, 2013; 8(4): e59243, Nat Biotechnol. 2014; 32(6): 554-61, Sci Rep. 2016; 6: 35680, or the like.

It can be determined whether or not a cell is a vascular endothelial cell by determining whether or not one or more vascular endothelial cell markers, such as TIE2, VEGFR-1, VEGFR-2, VEGFR-3, and CD41, are positive (to be determined as a vascular endothelial cell when positive). Besides, it can be determined whether or not a cell is a differentiated vascular endothelial cell by determining whether or not one or more markers, such as CD31 and CD144, are further positive (to be determined as a differentiated vascular endothelial cell when positive).

The term "mesenchymal cell" refers to a connective tissue cell that exists mainly in a connective tissue derived from the mesoderm and forms a support structure of a cell that functions in the tissue, and encompasses both concepts of a cell that has been differentiated (a differentiated mesenchymal cell) and a cell destined to differentiate into a mesenchymal cell but not yet differentiated into a mesenchymal cell (an undifferentiated mesenchymal cell), so-called a mesenchymal stem cell. A "vascular endothelial cell" is one of cells differentiating from an undifferentiated mesenchymal cell, but is excluded herein from the definition of the "mesenchymal cell".

It can be determined whether a cell is an undifferentiated mesenchymal cell or a differentiated mesenchymal cell by, for example, determining whether or not one or more undifferentiated mesenchymal cell markers, such as Stro-1, CD29, CD44, CD73, CD90, CD105, CD133, CD271, and Nestin, are positive (to be determined as an undifferentiated mesenchymal cell when positive, and determined as a differentiated mesenchymal cell when negative).

The mesenchymal cell may further express a cell marker specific to a specific organ (tissue) in accordance with the organoid to be obtained by the present invention or the "cell constituting an organ or a tissue" to be used in combination. Examples of the cell marker include FOXF1, COL4A, and ALCAM, which are cell markers for septum transversum mesenchyme (STM).

In one preferable embodiment of the present invention, the organoid is a kidney organoid (preferably, a kidney anlage), and the cells constituting an organ is cells constituting kidney (parenchymal cells and/or non-parenchymal cells of kidney). The kidney organoid and the kidney anlage can be produced by co-culturing a cell of kidney or metanephros with a vascular endothelial cell and a mesenchymal cell (preferably a mesenchymal stem cell) in accordance with a method described in, for example, WO2013/047639, WO2015/129822, or the like. The vascular endothelial cell and the mesenchymal cell to be used for producing the kidney organoid (kidney anlage) are the same as those used in producing the liver organoid (liver bud) described above.

In the "cell constituting kidney", the parenchymal cell of kidney (a renal glomerular endothelial cell, a glomerular epithelial cell, a proximal tubule cell, a loop of Henle cell, or a distal convoluted tubule cell) encompasses both concepts of a cell having been differentiated into a parenchymal cell (differentiated renal cell), and a cell destined to differentiate into a parenchymal cell but not yet differentiated (undifferentiated renal cell), that is, so-called a renal progenitor cell. The differentiated renal cell may be a cell collected from a living body (isolated from kidney in a living body), or may be a cell obtained by differentiating a pluripotent stem cell such as an ES cell or an iPS cell, a renal progenitor cell, or another cell having the ability to differentiate into a parenchymal cell of kidney. The undifferentiated renal cell may be a cell collected from a living body, or may be a cell obtained by differentiating a pluripotent stem cell such as an ES cell or an iPS cell, or another stem cell or progenitor cell. The cell capable of differentiating into a renal cell can be produced, for example, according to Morizane et al., Nat Protoc 12:195-207 (2017), Taguchi et al., Cell Stem Cell 14(1): 53-67 (2014), Narayanan et al., Kidney Int. 83(4): 593-603 (2013) or the like. A method for differentiating, into a renal cell, a pluripotent stem cell such as an ES cell or an iPS cell, a renal progenitor cell, or another cell having the ability to differentiate into a renal cell is known, and an iPS cell can be differentiated, for example, according to a method described in Morizane et al., Nat Protoc 12: 195-207 (2017), Taguchi et al., Cell Stem Cell 14(1): 53-67 (2014), Narayanan et al., Kidney Int. 83(4): 593-603 (2013) or the like as described above. For both of a cell population collected from a living body and a cell population produced by inducing differentiation of an ES cell, an iPS cell, or the like (particularly, for the latter), a cell population with high purity of a differentiated renal cell or a cell population with high purity of an undifferentiated renal cell may be used, or a cell mixture containing a differentiated renal cell and an undifferentiated renal cell at an optional ratio may be used.

It can be determined whether or not a cell is a differentiated renal cell by determining whether or not expression of one or more mature renal cell markers, such as PODXL, LTL, and CDH1, are positive. On the other hand, it can be determined whether or not a cell is an undifferentiated renal cell by determining whether or not expression of one or more cell markers, such as SIX2 and SALL1, are positive.

In one preferable embodiment of the present invention, the organoid has a vascular structure, and/or the cells constituting an organ coexists with vascular cells. The term "having a vascular structure" specifically refers to that a fine vascular structure is formed in the resultant organoid, in production of the organoid, by causing cells constituting the organ to coexist with vascular cells (by co-culturing).

Examples of the "vascular cell" include a vascular endothelial cell, a vascular smooth muscle cell, and a pericyte. For example, a liver organoid having a vascular structure can be produced by co-culturing liver endoderm cells (corresponding to undifferentiated cells of a liver parenchymal cell), vascular endothelial cells, and mesenchymal cells (preferably mesenchymal stem cells). Besides, a kidney organoid having a vascular structure can be produced by co-culturing cells of kidney or metanephros, vascular endothelial cells, and mesenchymal cells (preferably mesenchymal stem cells).

It can be determined whether or not an organoid has been obtained from cells constituting an organ, and the other cells used if necessary, by determining, by visual observation or microscopic observation, whether or not a conformational structure (a three-dimensional structure) has been formed. Besides, production of an organoid can be determined by, in addition to the formation of the conformational structure, determining whether or not a prescribed cell marker, and a marker of a parenchymal cell of the organ in particular, is positive (for example, whether or not a ratio of positive cells is over a prescribed level), and if necessary, whether or not a protein of the marker has been secreted into a culture supernatant (for example, whether or not the secretion amount is over a prescribed level). In production of, for example, a liver organoid or a liver bud, whether or not expression of a marker such as HHEX, SOX2, AFP, ALB or HNF4α is positive, and/or whether or not albumin (ALB) has been secreted into a culture supernatant can be employed for the determination. In production of a kidney organoid or a kidney anlage, whether or not expression of a marker such as SIX2, SALL1, PODXL, LTL, or CDH1 is positive can be employed for the determination.

### <Immobilization and Matrix>

In the culture method of the present invention, the organoid and/or the cells constituting an organ (the organoid or the like) is immobilized in a chamber. Means for "immobilization" is not especially limited, and any means may be used as long as the organoid or the like can be prevented from flowing out of the chamber by the culture fluid perfused to generate a turbulent flow in the chamber. For example, the whole or a part of the bottom of the chamber is covered with a matrix (the same as that used for embedding the organoid or the like, which will be described below), or a similar scaffold material or the like, the organoid or the like (prescribed cells used for producing the organoid) is seeded on such a covering material to be flat-cultured, and thus, the organoid or the like can be immobilized in the chamber. Alternatively, as in a preferable embodiment of the present invention described below, embedment of the organoid or the like in a matrix, for example, suspension of a matrix having the organoid or the like embedded therein from a breathable membrane, is also suitable means for immobilizing the organoid or the like in the chamber.

In one preferable embodiment of the present invention, the organoid and/or the cells constituting an organ (the organoid or the like) is immobilized in a state embedded in a matrix.

As the "matrix" for embedding the organoid or the like, a general extracellular matrix that is a solid at room temperature or more, and is used in cell culture, particularly in three-dimensional cell culture can be used. An extracellular matrix basically contains fibrous protein and proteoglycan as principal components, and examples of such components include elastin, entactin, osteonectin, collagen (type IV collagen), tenascin, thrombospondin, perlecan, vitronectin, fibrillin, fibronectin, heparin (sulfate), and laminin. For example, a basement membrane matrix known as a trade name "Matrigel" (Corning), containing laminin, collagen (type IV collagen) and entactin, and also containing growth factors such as EGF, IGF-1, PDGF, and TGF-β is one of preferable matrixes used in the present invention. Besides, a self-assembling peptide known as a hydrogel, such as hydrogels containing arginine, glycine and asparagine, can be used as the matrix of the present invention. As the matrix, one of these may be used, or two or more of these may be used in the form of a mixture, or without mixing (so as to form different layers when solidified).

Those skilled in the art can appropriately adjust the composition and concentration (undiluted or diluted) of the matrix so that the resultant matrix having the organoid or the like embedded therein (hereinafter referred to as the "matrix composition") have appropriate hardness. For example, the matrix (e.g., Matrigel) can be used in the form of a mixture with a culture fluid for improving handleability, and attaining appropriate hardness when solidified. In such an embodiment, the culture fluid to be mixed with the matrix can be the same as the culture fluid perfused in the culture method of the present invention, and used for culturing the organoid or the like.

The matrix composition can be produced generally by adding an appropriate amount of the matrix containing a suitable component to a culture fluid containing the organoid or the like, and solidifying the resultant matrix (with an ionic solution or an ionic molecule for solidifying a hydrogel added if necessary). For example, a matrix that is in a liquid state at 4°C or less (e.g., Matrigel) and the organoid or the like are mixed by stirring, the resultant is allowed to stand still at 37°C or more to solidify the matrix, and thus, the matrix composition is obtained. When the matrix is added to a comparatively large amount of medium in which the organoid or the like is suspended, a sufficient amount of the matrix is added to attain sufficient hardness of the matrix, and thus, the organoid or the like is properly held within the matrix without submerging onto the bottom.

The matrix composition may be any one of the following (i) to (iii):
(i) A matrix composition obtained by producing an organoid from prescribed cells, and isolating the organoid to be mixed with a matrix;
(ii) a matrix composition obtained by producing an organoid from prescribed cells by three-dimensional culture, and mixing the organoid with a matrix; and
(iii) a matrix composition obtained by adding a matrix to a cell population containing prescribed cells (cells constituting an organ) for producing an organoid, solidifying the resultant, and culturing the cell population in a state embedded in the matrix to produce the organoid.

Means for immobilizing the organoid and/or the cells constituting an organ (the organoid or the like) embedded in a matrix, namely, a matrix composition, within the chamber is not especially limited. The matrix composition may be disposed in a proper place within the chamber so that the perfused culture fluid can contact, in a turbulent flow state, with the matrix having the organoid or the like embedded therein. In the present invention, for example, a mixture of the organoid or the like and the matrix before solidification is dropped onto a proper place within a chamber, and the matrix is solidified, and thus, the organoid or the like can be immobilized within the chamber (in the state embedded in the matrix).

In one preferable embodiment of the present invention, the matrix having the organoid and/or the cells constituting an organ embedded therein (matrix composition) is immobilized in the chamber in a state suspended from a breathable membrane.

The term "breathable membrane" refers to a membrane having at least oxygen permeability, and further having, if necessary, permeability of carbon dioxide or another desired gas. Various breathable membranes are known, and examples include membranes made of fibers of polyethylene terephthalate (PET), polydimethylsiloxane (PDMS), fluorocarbon, polytetrafluoroethylene (PTFE), polyurethane and the like. The breathable membrane may be, if necessary, subjected to a surface treatment for increasing or reducing cell attachment, such as a coating treatment with an ECM of collagen or the like. Alternatively, the breathable membrane may be a membrane obtained by laminating a breathable membrane on a porous membrane (mesh) made of a fiber different from the breathable membrane (hybrid membrane), if necessary.

The matrix composition "in a state suspended from a breathable membrane" can be produced by dropping the matrix composition before solidification onto the breathable membrane, solidifying the resultant, and inverting the resultant in such a manner as to cause the solidified matrix composition attached to the breathable membrane to face downward (project downward). When the breathable membrane having the matrix composition suspended therefrom is immobilized within the chamber (for example, on a ceiling portion) with an appropriate member such as a holder, although the matrix composition is immersed in the perfused culture fluid, the breathable membrane is not immersed but placed in the air (or in a desired culture atmosphere), and thus, the organoid or the like embedded in the matrix can be cultured with gas exchange through the breathable membrane ensured.

### <Culture Fluid>

The culture fluid (liquid medium) to be perfused in the present invention may be suitably selected in accordance with the type of the organoid and/or the cells constituting an organ (the organoid or the like). A medium for the organoid or the like is known, and a mixture of a medium for culturing the cells constituting an organ used in the production of the organoid (usually a cell population containing a mixture of a plurality of types of cells) can be generally used. For example, when a liver organoid and/or cells constituting liver is to be cultured, a mixed medium of a medium for a hepatocyte and a medium for a vascular endothelial cell can be used, and when a kidney organoid and/or cells constituting kidney is to be cultured, a mixed medium of a medium for a renal cell and a medium for a vascular endothelial cell can be used.

Examples of a basal medium for a vascular endothelial cell include DMEM/F-12(Gibco), Stempro-34 SFM (Gibco), Essential 6 medium (Gibco), Essential 8 medium (Gibco), EGM (Lonza), BulletKit (Lonza), EGM-2 (Lonza), BulletKit (Lonza), EGM-2 MV (Lonza), VascuLife EnGS Comp Kit (LCT), Human Endothelial-SFM Basal Growth Medium (Invitrogen), and Human Microvascular Endothelial Cell Growth Medium (TOYOBO). Examples of an additive for a vascular endothelial cell include one or more selected from the group consisting of B27 Supplements (Gibco), BMP4 (bone morphogenetic protein 4), GSK β inhibitor (e.g., CHIR99021), VEGF (vascular endothelial cell growth factor), FGF2 (fibroblast growth factor (also designated as bFGF (basic fibroblast growth factor))), Folskolin, SCF (stem cell factor), TGF β receptor inhibitor (e.g., SB431542), Flt-3L (Fms-related tyrosine kinase 3 ligand), IL-3 (interleukin 3), IL-6 (interleukin 6), TPO (thrombopoietin), hEGF (recombinant human epithelial cell growth factor), hydrocortisone, ascorbic acid, IGF1, FBS (fetal bovine serum), an antibiotic (such as gentamicin or amphotericin B), heparin, L-glutamine, phenol red and BBE.

Examples of a basal medium for a hepatocyte include RPMI (Fujifilm Corporation) and HCM (Lonza). Examples of an additive for a hepatocyte include one or more selected from the group consisting of Wnt3a, activin A, BMP4, FGF2, FBS, HGF (hepatocyte growth factor), oncostatin M (OSM) and dexamethasone (Dex). To a medium for a hepatocyte, at least one selected from ascorbic acid, BSA-FAF, insulin, hydrocortisone and GA-1000 can be added if necessary. More specifically, examples of the medium for a hepatocyte include a medium obtained by removing hEGF (recombinant human epithelial cell growth factor) from HCM BulletKit (Lonza); and a medium obtained by adding 1% B27 Supplements (Gibco) and 10 ng/mL hHGF (Sigma-Aldrich) to PRMI1640 (Sigma-Aldrich). In particular, when a liver bud is to be produced, for example, a medium obtained by adding dexamethasone, oncostatin M and HGF to a medium obtained by mixing, in a ratio of 1:1, EGM BulletKit (Lonza) and HCM BulletKit (Lonza) from which hEGF (recombinant human epithelial cell growth factor) has been removed can be used.

Examples of a basal medium for a renal cell include ReproFF2 (REPROCELL), and StemFit Basic (Ajinomoto Co., Ltd.). An example of an additive for a renal cell includes FGF9. To a medium for a renal cell, one or more selected from the group consisting of CHIR and heparin can be added if necessary. More specifically, an example of the medium for a renal cell includes a medium obtained by adding 10 ng/mL FGF9 (R & D) to ReproFF2. In particular, when a kidney anlage is to be produced, for example, a medium obtained by adding 3 µM CHIR and 10 ng/mL FGF9 (R & D) to ReproFF2 can be used.

The viscosity of the culture fluid (liquid medium) is not particularly limited as long as the culture fluid can be perfused, and from the viewpoint that a turbulent flow is easily generated in the chamber, the viscosity is, for example, preferably 0.7 to 0.8 mPa·s under a culture condition (of, for example, about 37°C).

### <Culture Conditions and the like>

The other conditions for the culture method of the present invention, such as an atmosphere, a temperature, and a period, can be appropriately adjusted in accordance with the purpose of performing the culture method of the present invention, and embodiments of the organoid or the like. For example, for forming a conformational structure as an organoid of liver or kidney, or an organ bud (anlage) thereof from cells constituting the organ and the other cells used if necessary, the culture can be performed in 5% CO₂ at 30 to 40°C (preferably at about 37°C) for 1 to 10 days (for example, for 1 to 3 days for an organ bud (anlage)).

### <Drug>

In one preferable embodiment of the present invention, the culture fluid contains a drug. The "drug" is not especially limited as long as it acts on the organoid or the like, or it is used for analyzing whether or not it acts, and can be selected in accordance with the purpose. The "drug" is not especially limited as long as it can be used as an active ingredient of a medicament, such as a low molecular weight medicament, an antibody medicament, a peptide medicament, or a nucleic acid medicament. Alternatively, a substance used in transcatheter arterial chemoembolization for embolizing a blood vessel (such as a gelatin sponge, or porous gelatin granules) can be used as a drug to be added to the culture fluid, particularly to be contained in the culture fluid in a vascular structure of an organoid, or as a drug for a drug evaluation method using a conformational organ (that may be transplanted in a non-human animal) described below. The "drug" is not limited to a drug actually commercially available as a medicament, but may be a drug used in a clinical test or non-clinical test, or a drug under development at the previous stage of the test (a candidate of an active ingredient of a medicament).

For example, a drug for treating an ischemic disease (a disease involving ischemia), an infarction or necrosis disease, or a hemorrhagic disease (herein, these diseases being generically referred to as the "ischemic disease or the like") in each organ is one of preferable drugs in the present invention. To a culture fluid for an organoid or the like corresponding to each organ, a therapeutic drug for the ischemic disease or the like in the organ can be added to be cultured. Examples of the ischemic disease or the like in liver include ischemic liver disorder (ischemic hepatitis), ischemic bile duct disorder, and liver rupture (secondary hemorrhage due to cancer, injury, an infectious disease or the like). Examples of the ischemic disease or the like in kidney include ischemic renal disorder, and essential renal bleeding. An example of a therapeutic drug for ischemic renal disorder approved in Japan and the like includes eculizumab. Examples of the ischemic disease or the like in pancreas include ischemic acute pancreatitis, and ischemic chronic pancreatitis. Examples of the ischemic disease or the like (blood flow reduction or the like) in thyroid gland or parathyroid gland include subacute thyroiditis, and painless thyroiditis. An example of the ischemic disease or the like in heart includes ischemic heart disease. An example of a therapeutic drug for ischemic heart disease approved in Japan and the like includes isosorbide mononitrate. An example of the ischemic disease in brain includes cerebral ischemia.

In another aspect, a drug that may cause a side reaction involving ischemia or bleeding in an organ can be a preferable drug in the present invention as a drug for safety evaluation. Examples of a drug that may cause ischemia as a side reaction include an anticancer agent, an antiviral agent, and acetaminophen causing serious drug-induced liver injury in liver, and in addition, an antimicrobial (for multiple organs), a cardiac stimulant, a steroid (heart), a diuretic, a renin-angiotensin system (RAS) inhibitor, a contrast agent, and an SGLT2 inhibitor (kidney). Examples of a drug that may cause bleeding as a side reaction include an antiplatelet agent, and a thrombolytic drug.

### - Drug Evaluation Method -

The culture method of the present invention can be applied to a method for evaluating a drug by using a culture fluid containing the drug. A viewpoint for evaluating a drug is not especially limited, and for example, when the drug is used for treating an ischemic disease or the like in an organ, efficacy in the treatment can be evaluated. Alternatively, when the drug may cause a side reaction involving ischemia or bleeding in an organ, safety of the drug can be evaluated.

In other words, the drug evaluation method using the organoid or the like of the present invention includes a step of performing the culture method of the present invention by using a culture fluid containing any of the above-described drugs (hereinafter referred to as the "drug-added culture step"), and further includes a step of evaluating efficacy and/or safety of the drug against the organoid.

More specifically, a method for evaluating efficacy of a drug of the present invention (herein, also referred to as the "medicinal effect evaluation method") includes, for example, a drug evaluation method including the drug-added culture step performed by using a culture fluid containing a drug for treating an ischemic disease or the like in liver, kidney or the like. A method for evaluating safety of a drug of the present invention includes, for example, a medicinal effect evaluation method including the drug-added culture step performed by using a drug that may cause a side reaction involving ischemia or bleeding in an organ.

Culture conditions to be employed in the drug-added culture step (such as an atmosphere, a temperature, and a period) can be basically the same as the culture conditions employed in the culture method of the present invention, and can be appropriately adjusted if necessary, for example, a culture period sufficient for making evaluation suitable to the drug can be employed.

The drug evaluation method of the present invention may include another step in addition to the drug-added culture step if necessary. For example, the method can include a step of evaluating efficacy and/or safety of a drug by (i) detecting dead cells, from cells that have undergone the drug-added culture step, by using a reagent (such as propidium iodide; PI) to determine a proportion of dead cells in the cells (cell death rate), and/or (ii) measuring a production amount of a biomarker reflecting the symptom of a disease to be treated (such as a concentration of a specific substance in a culture supernatant) to be compared with a control.

In the drug evaluation method of the present invention, the organoid or the like is preferably derived from a patient (human) or another target having developed a disease to be treated with the drug. In this embodiment, the cells constituting an organ and the other cells used if necessary may be cells collected from such a patient or target (primary cultured cells), or may be iPS cells or subcultured cells (established cells) produced therefrom. For example, an iPS cell strain produced using a cell collected from a patient of an ischemic disease or the like is, in performing the drug evaluation method of the present invention as a standard method, suitable for producing an organoid, namely, for differentiation induction into cells constituting an organ and other cells used if necessary for producing an organoid.

The drug evaluation method of the present invention can be similarly performed also when a conformational organ obtained by a production method described below is a target instead of the organoid or the like (the organoid and/or the cells constituting an organ). For example, in one preferable embodiment of the present invention, a drug evaluation method using a conformational organ of the present invention can include a step of adding a drug to a medium for a conformational organ, and a step of evaluating efficacy (medicinal effect) and/or safety of the drug against the conformational organ.

The present invention also provides a drug evaluation method including a step of administering a drug to a non-human animal in which a conformational organ has been transplanted, and a step of evaluating efficacy and/or safety of the drug against the conformational organ.

### - Method for Producing Conformational organ -

A method for producing a conformational organ of the present invention includes a step of performing the culture method for an organoid or the like of the present invention described above. The term "conformational organ" refers to a structure that can be designated as a mature organoid, and contains a cell population or structure more mature than an organoid.

Culture conditions to be employed in the method for producing a conformational organ of the present invention (such as an atmosphere, a temperature, and a period) can be basically the same as the culture conditions employed in the culture method of the present invention, and can be appropriately adjusted if necessary, for example, a culture period sufficient for forming a conformational organ can be employed. For example, when a conformational organ of liver, kidney or the like is to be produced, culture is performed for 20 to 30 days from a day when culture of a prescribed cell for producing an organoid is started, or culture is performed for another 10 to 20 days for mature from a day when formation of a three-dimensional structure as an organoid can be confirmed.

It can be determined whether or not a conformational organ has been obtained from an organoid by making determination from one or more viewpoints such as a density of cells in the structure (whether or not the density is over a prescribed level), a conformational shape of the structure (whether or not the shape is more conformational beyond a prescribed level), function or trait (whether or not a prescribed function or trait, such as metabolic function, has been acquired), and a cell marker (whether or not expression of a gene or protein of a cell marker is positive, whether or not a density of positive cells is over a prescribed level, or whether or not a secretion amount of a marker protein in a culture supernatant is over a prescribed level). The density of the cells, the conformational shape, the function or trait, the cell marker and the like described above can be appropriately set in accordance with the organoid and the conformational organ, and for example, whether or not a level equivalent to or similar to that of an organ in a living body has been achieved can be employed as a criterion for the determination.

For example, when liver is to be produced as a conformational organ, acquisition of metabolic function such as production of glutamine and citric acid or gluconeogenesis, whether or not expression of a marker such as ASGR1 or drug metabolizing enzyme (CYP3A4 or CYP7A1) is positive, for example, whether or not ASGR1-positive cells are present in a density of 70% or more, can be used for the determination. When kidney is to be produced as a conformational organ, whether or not expression of some of or plurality of markers such as PODXL, LTL, and CDH1, pilus proteins (PKD1, PKD2, NPHP1, NPHP6, and PKHD1), transport proteins (SLC34A1, ATP1A1, SLC6A19, SLC9A3, SLC2A2, ABCB1, and LRP2) are positive can be used for the determination.

An artificial organ can be produced from a conformational organ by linkage to an extracorporeal circulation. Such an artificial organ can be used as an organ failure model, or can be used for evaluating the function of an organ. As for such applications, for example, WO2013/047720 and the like can be referred to.

### - Culture System -

A culture system of the present invention is a culture system including a "chamber" capable of holding an organoid and/or cells constituting an organ (organoid or the like) immobilized therein, and having an inlet and an outlet for perfusing a culture fluid for the organoid or the like, and "perfusion means" for perfusing the culture fluid, wherein the perfusion means is controlled to generate a turbulent flow in the culture fluid within the chamber.

The chamber is the same as that described above in relation to the "method for culturing an organoid or the like" of the present invention, and the culture system of the present invention uses such a chamber, or a culture vessel (cell culture device) provided with the chamber.

As the perfusion means of the culture system of the present invention, general or known means used for perfusion culture of an organoid or the like, or another tissue, cell or the like can be employed. The perfusion means representatively includes, as a basic structure, a pump (a peristaltic pump or the like) or another liquid feed means for feeding a culture fluid, a reservoir for reserving the culture fluid, and passages (tubes or the like) respectively linking between the reservoir and the pump or the like, between the pump or the like and the inlet of the chamber, and between the outlet of the chamber and the reservoir.

The perfusion means, particularly the liquid feed means such as a pump, is preferably controlled so that a flow rate or the like can be changed at an optional (precedently set) timing. For example, in one preferable embodiment of the present invention, the perfusion means is controlled to perfuse the culture fluid to have (i) time of transition from a laminar flow to a turbulent flow in the chamber, (ii) time of generation of a turbulent flow, and (iii) time of transition from a turbulent flow to a laminar flow. In this embodiment, the perfusion means can be controlled to have time (iv) of generation of a laminar flow (which may be static time) in addition to the times (i) to (iii), and to continuously repeat these times. For example, liquid feed at a first flow rate or the like for a prescribed time period, or no liquid feed for a prescribed time period (corresponding to a case of the first flow rate or the like being zero), and subsequent liquid feed at a second flow rate or the like for a prescribed time period are set as one cycle, and this cycle can be repeated a prescribed number of times of cycles (over a prescribed time period). It is noted that a control program for the perfusion means, particularly the liquid feed means such as a pump, can be a part of the culture system of the present invention.

The culture system of the present invention may include, if necessary, other means, structures and the like used in general or known culture systems for perfusion culture. An example of such means, structures and the like includes means for keeping a culture environment at a suitable temperature and atmosphere (a temperature control device or mechanism, and an atmosphere control device or mechanism, and the like).

### Examples

### [Example 1]

### [1] Production of Human Non-hemogenic Endothelial Cell

Human iPS cells (1383D2; Center for iPS Cell Research and Application, Kyoto University) were cultured in DMEM/F-12 (Gibco) (10 ml) supplemented with 1% B-27 Supplements (Gibco), BMP4 (25 ng/ml) and CHIR99021 (8 µM) in 5% CO₂ at 37°C for 3 days to induce mesoderm cells. The mesoderm cells thus obtained were further cultured in Stempro-34 SFM (Gibco) (10 ml) supplemented with VEGF (200 ng/ml) and Folskolin (2 µM) in 5% CO₂ at 37°C for 7 days to obtain a CD31-positive, CD73-positive and CD144-positive human non-hemogenic endothelial cell population.

### [2] Production of Human Liver Endoderm Cell

Human iPS cells (1383D2; Center for iPS Cell Research and Application, Kyoto University) were cultured in a basal medium, RPMI (Fujifilm Corporation) (2 ml) supplemented with Wnt3a (50 ng/mL) and activin A (100 ng/ml) in 5% CO₂ at 37°C for 5 days to induce endoderm cells. The endoderm cells thus obtained were cultured with 1% B27 Supplements (Gibco) and FGF 2 (10 ng/ml) added to the basal medium in 5% CO₂ at 37°C for another 5 days to obtain an AFP-positive, ALB-positive and HNF4α-positive human liver endoderm cell population.

### [3] Production of Human Mesenchymal Cell

Human iPS cells (1383D2; Center for iPS Cell Research and Application, Kyoto University) were cultured in a basal medium, DMEM/F-12 (Gibco) (10 ml) supplemented with 1% B-27 Supplements (Gibco), BMP4 (25 ng/ml) and CHIR99021 (8 µM) in 5% CO₂ at 37°C for 3 days to induce mesoderm cells. The mesoderm cells thus obtained were cultured with PDGFBB and activin A added to the medium in 5% CO₂ at 37°C for another 3 days. The cultured cells were collected and seeded again in a new gelatin-coated plate, and cultured with bFGF and BMP4 added to the basal medium in 5% CO₂ at 37°C for another 4 days to obtain a FOXF1-positive, COL4A-positive, ALCAM-positive and CD73-positive human mesenchymal cell population.

### [4] Cell Embedding in Extracellular Matrix and Production of Matrix Composition

A medium obtained by mixing a liver induction medium obtained by adding FBS, HGF, OSM and Dex to HCM (manufactured by Lonza) with EGM (Lonza) in a volume ratio of 1:1 (herein, referred to as the "organoid medium") was prepared. Besides, the organoid medium and Matrigel (BD Pharmingen) were mixed in a volume ratio of 1:1 to prepare a matrix (herein, referred to as the "organoid medium/Matrigel mixed matrix").

5 µL of the organoid medium/Matrigel mixed matrix was dropped onto an inverted Hanging Drop Insert (Millipore) and solidified by heating the resultant to 37°C, and thus, a "third matrix layer" was formed.

The human liver endoderm cell, the human non-hemogenic endothelial cell and the human mesenchymal cell produced as described above were mixed in a ratio of 10:7:1 with the organoid medium/Matrigel mixed matrix at 4°C. 3 µL of the thus obtained mixture was dropped onto the third matrix layer formed as described above, and the resultant was solidified by heating to 37°C, and thus, a "second matrix layer" was formed.

Subsequently, 8 µL of the organoid medium/Matrigel mixed solution was dropped onto the second matrix layer formed as described above, and the resultant was solidified by heating to 37°C, and thus, a "first matrix layer" was formed.

To a low-attachment 24-well plate, 600 µL of the organoid medium supplemented with a Rock (Rho-associated coiled-coil forming kinase) inhibitor (10 ng/mL) was added. A matrix composition containing the first to third matrix layers having been formed on the Hanging Drop Insert as described above was inserted toward the medium in the well. After 24 hours, the medium was exchanged by half with the organoid medium excluding a Rock inhibitor (10 ng/mL), and as medium exchange thereafter, a half of the medium was exchanged every day until day 3 after starting the culture.

### [5] Perfusion Culture

On day 3 after starting the culture, the Hanging Drop Insert to which the matrix composition containing the organoid had been attached was taken out, and perfusion culture was started using QuasiVivo QV600 Barrier Sys. Starter Kit (Kirkstall) including a culture chamber and a supply medium tank. As a pump for feeding a culture fluid, a tubing pump (530SN/313D) manufactured by Watson-Marlow Ltd. was used, and a Tygon tube (inner diameter of 8 mm x outer diameter of 11 mm) was connected as a pump-side tube. Feeding was performed at 20 mL/min for 30 seconds every 30 seconds for performing intermittent perfusion. Figure 1 is a schematic diagram of the apparatus. It was visually confirmed that a turbulent flow vortex was generated in the culture chamber during the perfusion (see Figure 2). The culture was performed in the organoid medium in 5% CO₂ at 37°C for 11 days, and medium exchange was performed by replacing the organoid medium held in the supply medium tank once three days.

On day 11 after starting the perfusion culture, the Hanging Drop Insert to which the matrix composition containing the organoid had been attached was taken out, and the organoid was scraped with a cell scraper. The resultant organoid was put and immobilized in a 2 mL tube containing a 4% paraformaldehyde/PBS immobilization solution.

### [6] Static culture (Control)

As a control, culture was performed for the same period using the matrix composition containing the organoid and the organoid medium the same as those described above by conventional general static culture, namely, without performing the perfusion culture from day 3 after starting the culture but by continuously performing the culture in the low-attachment 24-well plate, and the resultant organoid was immobilized in the same manner.

### [7] Immunostaining (ASGR1 or the like)

After removing the immobilization solution, the resultant was washed with PBS (15 minutes x twice), followed by a permeation treatment with 20% DMSO/ethanol, 80% ethanol, and 50% ethanol respectively for 15 minutes. Besides, immunostaining was performed with an anti-ASGR1 antibody (R & D, MAB4394), an anti-CD31 antibody (Abeam, AB28364), fluorescence labeling secondary antibodies binding to these antibodies (Novex Donkey anti-Mouse IgG (H + L) Secondary Antibody, Novex Donkey anti-Rabbit IgG (H + L) Secondary Antibody (Invitrogen)), and a nuclear staining reagent (DAPI), followed by a transparency treatment with Visikol HISTO-M (Visikol Inc.). The thus obtained stained specimen was subjected fluorescence image observation with LSM 880 Confocal Laser Microscope (Zeiss), a binarized ASGR1 fluorescent region area was created based on the thus obtained fluorescence image, and an ASGR1 positive rate was calculated by dividing the resultant by a similarly binarized DAPI fluorescent region area. Results are illustrated in Figure 3.

### [Example 2]

The human liver endoderm cell, the human non-hemogenic endothelial cell and the human mesenchymal cell produced in the same manner as described in [1] to [3] of Example 1 were used to obtain an organoid by perfusion culture of a matrix composition in the same manner as described in [4] and [5] of Example 1, and the resultant organoid was immobilized. Besides, as a control, an organoid was obtained by static culture of a matrix composition in the same manner as described in [6] of Example 1, and the resultant organoid was immobilized. Observed images (three-dimensional structures) of the matrix compositions containing the organoids respectively obtained by the perfusion culture and the static culture are illustrated in Figure 4.

### [8] Immunostaining (GS, E-cadherin and the like)

After removing the immobilization solution, the resultant was washed with PBS (15 minutes x twice), followed by a permeation treatment with 20% DMSO/ethanol, 80% ethanol, and 50% ethanol respectively for 15 minutes. Besides, immunostaining was performed with an anti-glutamine synthase (GS) antibody (Abeam, ab73593), an anti-E-cadherin antibody (Abeam, ab76055), fluorescence labeling secondary antibodies binding to these antibodies (Novex Donkey anti-Mouse IgG (H + L) Secondary Antibody, Novex Donkey anti-Rabbit IgG (H + L) Secondary Antibody (Invitrogen)), and a nuclear staining reagent (DAPI), followed by a transparency treatment with Visikol HISTO-M (Visikol Inc.). The thus obtained stained specimen was subjected fluorescence image observation with LSM 880 Confocal Laser Microscope (Zeiss) to obtain fluorescence observed images. Results are illustrated in Figure 5.

## Claims

1. A culture method for culturing an organoid and/or cells constituting an organ immobilized in a chamber with a culture fluid perfused,
wherein the culture fluid is perfused in such a manner as to generate a turbulent flow in the chamber.

2. The culture method according to claim 1, wherein the culture fluid is perfused in such a manner as to have time of transition from a laminar flow to a turbulent flow, time of generation of a turbulent flow and time of transition from a turbulent flow to a laminar flow in the chamber.

3. The culture method according to claim 1, wherein the organoid and/or the cells constituting an organ is immobilized in a state embedded in a matrix.

4. The culture method according to claim 1, wherein a matrix in which the organoid and/or the cells constituting an organ is embedded is immobilized in the chamber in a state suspended from a breathable membrane.

5. The culture method according to claim 1, wherein the organoid has a vascular structure, and/or the cells constituting an organ coexists with vascular cells.

6. The culture method according to claim 1, wherein the organoid is a liver or kidney organoid, and/or the organ is liver or kidney.

7. A production method for producing a conformational organ, comprising a step of performing the culture method according to any one of claims 1 to 6.

8. The culture method according to any one of claims 1 to 6, wherein the culture fluid contains a drug.

9. The culture method according to claim 8, wherein the drug is for treatment of a disease involving ischemia.

10. A medicinal effect evaluation method, comprising a step of performing the culture method according to claim 8 or 9.

11. An organoid obtained by the culture method according to any one of claims 1 to 6.

12. A conformational organ obtained by the production method according to claim 7.

13. A medicinal effect evaluation method, comprising a step of adding a drug to a medium of the conformational organ according to claim 12, and a step of evaluating a medicinal effect of the drug on the conformational organ.

14. A culture system, comprising:
a chamber capable of holding an organoid and/or cells constituting an organ immobilized therein, and including an inlet and an outlet for perfusing a culture fluid for the organoid and/or the cells constituting an organ; and
perfusion means for perfusing the culture fluid,
wherein the perfusion means is controlled in such a manner as to generate a turbulent flow in the culture fluid in the chamber.

15. The system according to claim 14, wherein the perfusion means is controlled to perfuse the culture fluid in such a manner as to have time of transition from a laminar flow to a turbulent flow, time of generation of a turbulent flow and time of transition from a turbulent flow to a laminar flow in the chamber.
